Europäisches Patentamt

European Patent Office

Office européen des brevets

(19)

(11) Publication number: **0 017 656**
**B1**

(12) **EUROPEAN PATENT SPECIFICATION**

(45) Date of publication of patent specification: 08.12.82

(21) Application number: 79101207.3

(22) Date of filing: 20.04.79

(51) Int. Cl.³: **C 12 N 9/92**, C 12 N 1/20,
C 12 P 19/24, C 07 B 29/00
//C12R1/045

(54) Process for producing glucose isomerase from ampullariella, process for isomerizing D-glucose to D-fructose, and cultures containing new species of ampullariella.

(43) Date of publication of application:
29.10.80 Bulletin 80/22

(45) Publication of the grant of the patent:
08.12.82 Bulletin 82/49

(84) Designated Contracting States:
DE FR GB IT NL

(56) References cited:
FR - A - 2 202 156

BIOSIS ARTICLE BIOLOGICAL ABSTRACTS
59041048 C. S. JUAN et al.: "A taxonomic
Study of Actinoplanaceae part 1 Classification
of Ampullariella", pages 31—41

BIOSIS ARTICLE BIOLOGICAL ABSTRACTS
58000719 T. W. JOHNSON: "Aquatic fungi of
Iceland some Kerathinophilic Species", pages
293—302

(73) Proprietor: THE DOW CHEMICAL COMPANY
Dow Center 2030 Abbott Road Post Office Box
1967
Midland Michigan 48640 (US)

(72) Inventor: Foley, Sharonkay Evans
7995 E. Mississippi No. J-6
Denver, Colorado 80237 (US)
Inventor: Oriel, Patrick John
3110 McKinley Road
Midland Michigan (US)
Inventor: Epstein, Carol Cherkis
5201 Nakahoma Drive
Midland Michigan (US)

(74) Representative: Casalonga, Axel et al,
BUREAU D.A. CASALONGA OFFICE JOSSE &
PETIT Baaderstrasse 12-14
D-8000 München 5 (DE)

**0 017 656**

Process for producing glucose isomerase from ampullariella, process for isomerizing
D-Glucose to D-fructose, and cultures containing new species of ampullariella

This invention concerns the production of a heat stable glucose isomerase from a microorganism belonging to the genus *Ampullariella*, a method for using the isomerase to convert glucose to fructose, and cultures containing new species of *Ampullariella*.

Glucose and fructose are the monosaccharides which are liberated upon the hydrolysis of sucrose. Glucose, also known as dextrose, is an aldohexose which is produced commercially from starch, especially corn starch, by the food processing industry. Fructose, also called levulose, is a ketohexose which is not present in significant concentrations in starch-derived syrups and sugars. However, because fructose is sweeter than glucose, it is desirable to convert at least part of the glucose in the starch-derived syrup or sugar to fructose to give the product the taste characteristics of cane or beet sugar. The isomerization of glucose to fructose may be accomplished by the use of various alkaline catalysts or by means of an enzymatic conversion. Alkaline catalysis, although widely used in the past, gives rather low yields and also produces undesirable by-products which affect the quality of the syrup. As a result of these drawbacks, enzymatic conversions using a glucose isomerase have become increasingly popular.

A number of glucose isomerases have been isolated from different species of microorganisms. Glucose isomerase has been produced by microorganisms from the generae *Streptomyces*, *Actinoplanes*, *Bacillus*, *Flavobacterium*, *Brevibacterium*, *Arthrobacter*, *Nocardia*, *Micromonospora*, *Microbispora*, and *Microellobospora*. Disadvantages of using an enzyme conversion of glucose to fructose reside in the cost of producing the enzyme and the sensitivity of the enzyme to environmental parameters. Isomerase preparations are usually inactivated by higher temperatures, so relatively low temperatures must be used to carry out the isomerization. Thus, longer overall reaction times are necessary than would be required if the isomerization could be carried out at a higher temperature. In addition, immobilization techniques necessary to recover the isomerase during processing are limited to those employing only mild temperature conditions. Therefore, a glucose isomerase having good heat stability is especially preferred.

French Patent 2.202.156 (Anheuser Bush) discloses namely the production of glucose isomerase by various species of Actinoplasnes and namely as particularily preferred, the species Actinoplanes missouriensis. Although it is stated that the glucose isomerase is active at temperatures up to 90°C, the glucose isomerase produced by this microorganism still lacks however sufficient long-term heat-stability.

The present invention is directed to a method of producing glucose isomerase from a microorganism belonging to the genus *Ampullariella* which comprises growing the *Ampullariella* in a culture medium and recovering the glucose isomerase therefrom. The glucose isomerase produced using this method has demonstrated superior thermal stability. Samples of the isomerase derived from some strains have been shown to retain essentially all of their original activity after heating at 75°C for a period of 24 hours.

The present invention is also directed to a process for converting D-glucose to D-fructose which includes the step of treating an aqueous solution containing glucose with an isomerase produced by a microorganism belonging to the genus *Ampullariella* thereby converting a portion of the glucose to fructose.

Due to the difficulty of isolating members of the genus only four species of *Ampullariella* had been previously described in the literature. See *Jour. of the Mitchell Soc.*, May 1963, page 53. Known species include *Ampullariella lobata*, *Ampullariella digitata*, *Ampullariella campanulata*, and *Ampullariella regularis*. According to this invention, it has been found that all the known species produce glucose isomerase. In addition, new organisms belonging to the genus have been isolated and found to produce glucose isomerase. These isolates although belonging to the genus *Ampullariella* cannot be placed into any of the known species within the genus, and therefore represent previously unknown species of *Ampullariella*. These novel isolates are referred to herein as *Ampullariella* species 3876 (ATCC 31351), *Ampullariella* species 3877 (ATCC 31352), *Ampullariella* species 3965 (ATCC 31353), and *Ampullariella* species 3966 (ATCC 31354). Especially preferred for the production of glucose isomerase is the novel species *Ampullariella* 3876, the enzyme of which has demonstrated superior heat stability. Mutant microorganisms derived from the species disclosed herein and capable of producing glucose isomerase are considered as being within the scope of the invention. Subcultures of the previously unknown isolates have been made part of the permanent collection of the American Type Culture Collection, 12301 Parklawn Drive, Rockville, Maryland. The date of deposit of the cultures was December 7, 1977.

The genus *Ampullariella* is characterized as having rod-shaped zoospores, 0.3—0.7 by 0.8 to 1.5 $\mu$m, with a single polar flagellum or from one to two lateral flagella. The agar colonies are generally yellow-brown, yellow-grey, yellow or bright lemon yellow. The most distinctive feature of the genus are the predominantly cylindrical sporangia and the zoospores which are generally arranged end to end in longitudinal rows within the sporangia. Normally, the organisms grow saprophytically on a variety of plant and animal material, especially on hair and other keratinous matter. They are found in soils.

2

**0017656**

*Ampullariella* are aerobic and grow well on various nutrient medium which contain an assimilable source of nitrogen, an assimilable source of carbon, and essential minerals. Although for the production of glucose isomerase it is preferred that the culture medium contain xylose, the isomerase will be produced in lesser amounts in the absence of xylose. The amount of xylose required in the medium to give satisfactory enzyme production for commercially recoverable quantities is generally between about 0.1 percent and 10 percent by weight. Members of the genus generally grow best at temperatures between about 15 and 45°C. The glucose isomerase is produced intracellularly and is generally not excreted into the surrounding culture medium. To isolate the isomerase it is, therefore, necessary to disrupt the cell walls to release the enzyme. Disruption of the cell walls may be accomplished by techniques well known in the art, such as, for example, by ultrasonic rupture.

As recognized by one skilled in the art, the conversion of D-glucose to D-fructose using the glucose isomerase produced above is generally carried out in an aqueous medium. Aqueous solutions useful in the isomerization of glucose, as for example corn syrup, may contain from about 5 percent to 80 percent glucose by weight, with about 30 percent to 60 percent being preferred. The final fructose concentration of the isomerized solution will depend on a number of factors well understood in the art, such as for example the amount of glucose isomerase used, the period of treatment, the temperature employed, pH of the solution, etc. In addition, as the equilibrium between the aldo and keto hexoses is approached, the rate of isomerization will decrease; therefore, the process is generally terminated when the percent weight of fructose is between about 40 to 50 percent of the total combined dry weight of glucose and fructose.

Pursuant to the present invention, the syrup may be treated with glucose isomerase using either a batch or continuous process. In either case, it is generally preferred that the enzyme be immobilized by a water-insoluble support, usually a polymer, either by physical absorption covalent bonding, or by entrapment. In the first two methods of immobilization, a glucose isomerase/water-insoluble polymer conjugate is formed which makes retention and recycling of the enzyme possible. Conjugates formed by the covalent bonding of the isomerase to the water-insoluble polymer include conjugates resulting from the reaction between the isomerase and a water-insoluble, functionalized polymer; the incorporation of the isomerase into a growing polymer chain; or the intermolecular cross-linking of the enzyme with a multifunctional, low molecular weight reagent. Synthetic supports which have been used for covalently bonding to enzymes and which would be suitable for use with the present invention include polymers based upon acrylamide, maleic anhydride, methacrylic acid, and styrene. Well known natural supports include agarose, cellulose, dextran and starch. Commonly used adsorbents which have been used to immobilize enzymes include alumina, ion-exchange resins, calcium carbonate, carbon, celluloses, clays, collagen, collodion, conditioned metal or glass surfaces, diatomaceous earth, and hydroxyl-apatite. Various methods of forming such glucose isomerase/water-insoluble polymer conjugates are discussed in texts such as *Immobilized Enzymes*, by O. Zaborky (CRC Press, Ohio) 1973, See also U.S. Patents 3,519,538; 3,788,945; 3,933,587; and 3,933,589.

Another suitable method for the immobilization of the glucose isomerase is by use of a hollow fiber reactor. This type of system may be preferable to immobilization on an active support where the possibility of active chemical residues should be avoided, as for example in the production of foodstuffs. See *Jour. of Food Science 42*, 258 (1977).

The amount of glucose isomerase employed in the isomerization of the syrup can vary considerably. Although greater or lesser amounts of glucose isomerase may be used in carrying out the method of this invention, in practice productivity ranges of between about 50 pounds and 5000 pounds (kg) of fructose/glucose mixture as dry solids per pound (kg) of immobilized glucose isomerase are preferred. As used herein, one glucose isomerase unit is defined as the amount of glucose isomerase that will convert one micromole of glucose to fructose per minute in a solution containing two moles of glucose/liter, 0.02 moles of magnesium sulfate/liter, and 0.001 mole of cobalt chloride/liter at a pH of 6.85 (0.2 M sodium maleate) at 60°C. Assay for D-glucose isomerase is done at 70°C with 5 percent D-glucose solution containing 2 mM cobalt chloride hexahydrate ($CoCl_2 \cdot 6H_2O$) and 50 m, magnesium sulfate heptahydrate ($MgSO_4 \cdot 7H_2O$). After appropriate dilution (0—50 $\mu g$ fructose/ml), the fructose concentration produced by the isomerase is determined by the cysteine-carbazole reaction at 60°C for 10 minutes. See *J. Biol. Chem. 192*, 583 (1951) and *Science 125*, 648 (1957). A control stopped with 7 percent perchlorate is run to determine background levels of glucose in the sample.

The conditions under which the isomerization may be carried out may show wide variation, although operable temperature and pH ranges generally vary from about 45° to about 85°C and from about pH 6 to about 8.5, respectively; more preferably the process is carried out at a temperature of from about 50° to about 75°C and at a pH of from about 6.5 to about 7.5.

Thermal stabilizing agents may be added to the syrup to inhibit the inactivation of the isomerase at the elevated temperature used to improve the efficiency of the process. Multivalent cations which are commonly used as thermal stabilizers and/or cofactors include cobalt, magnesium, and manganese ions. Optimal quantities of these ions may be readily determined by comparing the optimal temperature for isomerization to different concentrations of the thermal stabilizer and/or cofactor followed by an assay to determine the percent retention of isomerase activity after a given time period.

The present invention is further illustrated with the following examples; however these examples

3

**0 017 656**

should not be interpreted as a limitation upon the scope of the present invention.

Example 1

As noted above, a new species of *Ampullariella*, named *Ampullariella* species 3876 ATCC 31351 has been discovered. This species along with strains from the known species within the genus are capable of producing glucose isomerase. *Ampullariella* species 3876 is characterized morphologically by sporangia with irregular contours having a shape varying from oval to bottle shaped which vary in size from about 9 to 11 microns wide to about 14 to 18 microns long. Aerial mycelia are always absent; however, in some media the sporangial mass takes up the appearance of an aerial mycelium.

The cultural properties of *Ampullariella* species 3876 ATCC 31351 on different media suggested by Shirling and Gottlieb in *Int. J. Syste. Bacterial. 16,* pages 313—340 (1966) and Waksman in the *Actinomycetes,* Vol. 2 (Williams and Wilkins Co., Baltimore 1966) pages 328—334 are shown in Table 1. The cultural characteristics were determined after 6 to 14 days of incubation at 30°C.

TABLE I

The Number Accompanying Some of the Culture Media
Refers to Those Given by Shirling and Gottlieb

| Culture Media | Cultural Characteristics |
| --- | --- |
| Medium n° 2 (yeast extract — malt agar) | Abundant growth, crusty surface orange to 10 E 6* |
| Medium n° 3 (oatmeal agar) | Moderate growth, crusty surface orange light |
| Medium n° 4 (inorganic salts starch agar) | Abundant growth, crusty surface orange |
| Medium n° 5 (glycerol asparagine agar) | Abundant growth, smooth surface light orange 10 J 7* |
| Medium n° 6 (peptone yeast extract iron agar) | Moderate growth, smooth surface brown, deep brown pigment |
| Medium n° 7 (tyrosine agar) | Abundant growth, crusty surface orange, light brown pigment |
| Oatmeal agar (according to Waksam) | Abundant growth, wrinked surface orange 10 I 8* yellowish pigment |
| Hickey and Tresner's agar | Moderate growth, wrinkled surface brown, light brown pigment |
| Czapek glucose agar | Abundant growth, crusty surface orange 11 E 6* |
| Glucose asparagine agar | Abundant growth, crusty surface orange 9 K 9* |
| Nutrient agar | Moderate growth, smooth surface brown, light brown pigment |
| Potato agar | Abundant growth, wrinkled surface brown, brown pigment |
| Bennett's agar | Abundant growth, wrinkled surface amber brown |
| Calcium malate agar | Very scant growth, smooth surface hyaline |
| Skim milk agar | Abundant growth, wrinkled surface brown 15 C 12* |

TABLE I (cont.)

| Culture Media | Cultural Characteristics |
| --- | --- |
| Czapek agar | Scant growth, smooth surface light orange |
| Egg agar | Moderate growth, smooth surface light orange |
| Peptone glucose agar | Moderate growth, crusty surface orange to reddish 4 H 10* brown pigment |
| Agar | Very scant growth, hyaline |
| Loeffler serum | Very scant growth |
| Potato | Abundant growth, wrinkled surface orange to brown, brown pigment |
| Gelatin | Scant growth, cream to light orange |
| Cellulose agar | Very scant growth, thin, hyaline |

* Index numbers refer to *A Dictionary of Color,* edited by A. Maerz
and M. Rea Paul (McGraw-Hill, 1950).

Table II compares the ability of *Ampullariella* species 3876 ATCC 31351 and the known species to utilize carbon sources according to the method of Pridham et al. (*Intern. J. Syst. Bact. 56,* 107, 1948). Table III compares physiological characteristics of the various species of *Ampullariella*.

From the morphological characteristics (namely the predominantly cylindrical sporangia, the rod-shaped spores, and the arrangement of the spores end to end in longitudinal rows within the sporangium) strain ATCC 31351 is recognized as a member of the genus *Ampullariella*. However, the cultural characteristics, the pattern of pigmentation on different agars, the physiological characteristics (namely failure to form $H_2S$, to reduce nitrate and ability to peptonize litmus milk) and the pattern of carbon compound utilization (namely the ability to grow on the complex carbohydrate raffinose) separate this strain from any of the previously known species.

TABLE II

| MINIMAL MEDIUM CARBON COMPOUND UTILIZATION | Species 3876* | A. digitata* | A. lobata* | A. campanulata* | A. regularis* |
|---|---|---|---|---|---|
| Inositol | — | No growth | No growth | — | — |
| Fructose | + | " | " | — | + |
| Rhamnose | + | " | " | + | + |
| Mannitol | + | " | " | + | — |
| Xylose | + | " | " | + | + |
| Raffinose | + | " | " | — | — |
| Arabinose | + | " | " | + | + |
| Cellulose | — | " | " | — | — |
| Sucrose | + | " | " | — | + |
| Glucose | + | " | " | + | + |
| Mannose | + | " | " | + | + |
| Lactose | + | " | " | — | + |
| Salicin | + | " | " | — | + |

*+ represents growth  
— represents no growth

TABLE III

| CULTURAL CHARACTERISTICS | Species 3876 | A. digitata | A. lobata | A. campanulata | A. regularis |
|---|---|---|---|---|---|
| Vegetative mycelium | orange to brown (some media) | brown | orange | deep orange (brown in Czapek agar) | orange |
| Pigment | brown on various agar | brown (N.6—7 agar) | absent | absent | yellow (oatmeal agar) |
| **PHYSIOLOGICAL CHARACTERISTICS*** | | | | | |
| Hydrolysis of starch | + | + | + | + | + |
| $H_2S$ formation | — | + | + | + | + |
| Melanin production | + | + | — | — | — |
| Tyrosine reaction | — | — | — | — | — |
| Casein hydrolysis | — | — | + | — | — |
| Solubilization of calcium malate | — | — | — | — | — |
| Nitrate reduction | — | + | + | + | + |
| Liquefaction of gelatin | ± | ± | — | — | — |
| Litmus milk { coagulation | — | + | — | — | — |
| Litmus milk { peptinization | + | — | — | — | — |
| Cellulose decomposition | — | — | — | — | — |

*+ strongly positive; — strongly negative; ± weakly positive

**0 017 656**

In addition to *Ampullariella* species 3876, three other new isolates which each represent new species within the genus have been found to produce glucose isomerase. These isolates are designated as *Ampullariella* species 3877 (ATCC 31352), *Ampullariella* species 3965 (ATCC 31353), and *Ampullariella* species 3966 (ATCC 31354).

The utilization of carbon sources for each of these above species is shown in Table IV. The physiological characteristics of the new species are shown in Table V.

TABLE IV

| MINIMAL MEDIUM CARBON COMPOUND UTILIZATION | *Ampullariella* Species | | |
|---|---|---|---|
| | 3877* | 3965* | 3966* |
| Inositol | — | — | — |
| Fructose | + | + | + |
| Rhamnose | + | + | + |
| Mannitol | + | + | + |
| Xylose | + | + | + |
| Raffinose | — | — | + |
| Arabinose | + | + | + |
| Cellulose | — | — | — |
| Sucrose | + | + | + |
| Glucose | + | + | + |
| Mannose | + | + | + |
| Lactose | — | + | + |
| Salicin | + | + | + |

*+ represents growth
— represents no growth

8

# 0017 656

## TABLE V

| PHYSIOLOGICAL CHARACTERISTICS* | Ampullariella Species | | |
|---|---|---|---|
| | 3877 | 3965 | 3966 |
| Hydrolysis of starch | + | + | + |
| H₂S formation | + | + | + |
| Melanin production | + | + | + |
| Tyrosine reaction | — | — | — |
| Casein hydrolysis | — | — | — |
| Solubilization of calcium malate | — | — | — |
| Nitrate reduction | — | — | — |
| Liquefaction of gelatin | ± | ± | ± |
| Litmus milk { coagulation | — | — | — |
| Litmus milk { peptonization | + | — | — |
| Cellulose decomposition | — | — | — |

*+ represents strongly positive
— represents strongly negative
± represents weakly positive

## Example 2

A nutrient broth generally suitable for the maintenance of *Ampullariella* cultures is as follows:

| | |
|---|---|
| 3 grams | beef extract |
| 5 grams | peptone |
| 1 liter | distilled water |

For the production of glucose isomerase, an Emerson xylose growth media was found to be satisfactory. The ingredients are as follows:—

| | |
|---|---|
| 4.0 grams | beef extract |
| 4.0 grams | peptone |
| 2.5 grams | sodium chloride |
| 1.0 gram | yeast extract |
| 10.0 grams | xylose |
| 1.0 liter | distilled water |

## Example 3

Two ml of a nutrient broth culture of *Ampullariella* species 3966 was used as inoculum in a 500 ml baffled flask containing 100 ml of Emerson xylose growth medium.The culture was incubated three days at 30°C on a shaker. The cells were harvested by centrifugation at 16,300x g for 3 minutes and washed with 100 ml of 0.013 M phosphate buffer, pH 7.0. The cells were centrifuged a second time at 27,000x g for 30 minutes. The button of wet cells was resuspended in 0.05 M Tris-HCl buffer, pH 7.4 at a concentration of 10 ml buffer per gram of wet cells. The cells were ruptured at a temperature of 4°C using a Branson Sonifier[R] Cell Disruptor producing two, three minute pulses with a three-minute

9

rest between pulses. Cellular debris was removed by centrifugation at 27,000x g for 30 minutes. A sample of the crude extract was assayed for glucose isomerase activity by treating a 5% glucose solution with the extract and testing for the presence of fructose using the method described hereinbefore. The extract was found to contain 0.69 glucose isomerase units per milliliter of extract.

A second sample of the extract was heated for 24 hours at 75°C. Assay of the heated sample showed 0.54 glucose isomerase units per milliliter indicating the extract retained about 78 percent of the original glucose isomerase activity of the unheated control.

Example 4

*Ampullariella* species 3876 (130 wet grams) grown in a 14-liter New Brunswick fermenter with Emerson-xylose medium was harvested by centrifugation. The cells were washed with distilled deionized water and centrifuged at 4°C, 27,000x g for 30 minutes. The pellet of wet cells was resuspended in 0.025 m Tris-HCl buffer, pH 7.4 containing 0.5 mM cobalt chloride at a concentration of 5 ml buffer per gram of wet cells. The cells were ruptured at 4°C using a Branson Sonifier[R] Cell Disruptor at 50 watts to produce five three-minute pulses with a three-minute rest between pulses. Cellular debris was removed by centrifugation at 27,000x g for 30 minutes. A sample of the crude extract was assayed for glucose isomerase activity and protein concentration. The sample was found to contain 1.59 glucose isomerase units per milliliter of extract, and 5.6 mg protein per milliliter of extract (0.283 glucose isomerase units per milligram protein).

Crude glucose isomerase was concentrated two-fold by rotary evaporation at 48°C and purified two-fold (to 0.580 glucose isomerase units per milligram protein) by heating at 70°C for one hour at 150 rpm; centrifugation at 78,480x g, 4°C for 30 minutes removed precipitated protein from the isomerase-containing supernatant. Exhaustive dialysis of the isomerase against 0.05 M Tris-HCl, pH 7.4 was done to remove cobalt from the enzyme.

Assay of the final enzyme product using the method described in Example 3 above, showed 2.67 glucose isomerase units per milliliter. Samples of the enzyme which were heated at 75°C for 6 and 24 hours retained 112 percent and 104 percent, respectively, of their original activity as compared to a glucose isomerase unheated control.

## Claims

1. A process for producing glucose isomerase from a microorganism belonging to the genus *Ampullariella* which comprises growing the *Ampullariella* in a culture medium and recovering the gluclose isomerase therefrom.

2. The process of Claim 1 wherein the culture medium contains xylose or a xylose supplying source.

3. The process of Claim 1 or 2 wherein the microorganism is selected from *Ampullariella digitata, Ampullariella lobata, Ampullariella campanulata, Ampullariella regularis,* or a mutant thereof.

4. The process of Claim 1 or 2 wherein the microorganism is selected from *Ampullariella* species 3876 (ATCC 31351) *Ampullariella* species 3877, (ATCC 31352) *Ampullariella* species 3965, (ATCC 31353) *Ampullariella* species 3966, (ATCC 31354) or a mutant thereof.

5. A process for isomerizing D-glucose to D-fructose including the step of treating the D-glucose in an aqueous medium with an isomerase produced by a microorganism belonging to the genus *Ampullariella.*

6. The process of Claim 5 wherein a thermal stabilizing agent is present during the isomerization.

7. The process of Claim 6 wherein the thermal stabilizing agent is a cation selected from the group consisting of cobalt, magnesium, and manganese ions.

8. The process of Claim 7 wherein the glucose isomerase is immobilized on a water-insoluble support.

9. The process of Claim 8 wherein about one pound (kg) of immobilized glucose isomerase is present for the isomerization of from about 50 to 5000 pounds (kg) of glucose/fructose mixture as dry solids.

10. The process of Claim 9 wherein the pH is from about 6 to about 8.5 and the temperature is from about 45°C to about 85°C.

11. A biologically pure culture of a microorganism selected from *Ampullariella* species 3876, 3877, 3965, or 3966 having the identifying characteristics of ATCC 31351, 31352, 31353, or 31354, respectively, said culture being capable of producing glucose isomerase in a recoverable quantity upon fermentation in an aqueous nutrient media containing assimilable sources of carbon, nitrogen, and essential minerals.

12. A culture comprising a microorganism selected from *Ampullariella* species 3876 ATCC31351, 3877 ATCC31352, 3965 ATCC31353 or 3966 ATCC31354 mutant thereof and an aqueous nutrient culture medium containing assimilable sources of carbon, nitrogen, and essential minerals, said culture being capable of producing glucose isomerase in a recoverable quantity upon aerobic fermentation.

## 0017656

13. The culture of Claim 12 further including from about 0.1 percent to 10 percent by weight of xylose.

### Revendications

1. Procédé pour la production de glucose-isomérase à partir d'un micro-organisme appartenant au genre *ampullariella* qui comprend le développement de l'*ampullariella* dans un milieu de culture et la récupération de la glucose-isomérase à partir de ce milieu.

2. Procédé selon la revendication 1, dans lequel le milieu de culture contient du xylose ou une source fournissant du xylose.

3. Procédé selon la revendication 1 ou 2 dans lequel le micro-organisme est choisi parmi *Ampullariella digitata, Ampullariella lobata, Ampullariella campanulata, Ampullariella regularis,* ou un mutant de ces micro-organismes.

4. Procédé selon les revendications 1 ou 2, dans lequel le micro-organisme est choisi parmi *Ampullariella* espèce 3876 (ATCC 31351), *Ampullariella* espèce 3877 (ATCC 31352), *Ampullariella* espèce 3965 (ATCC 31353), *Ampullariella* espèce 3966 (ATCC 31354), ou un mutant de ces espèces.

5. Procédé d'isomérisation du D-glucose en D-fructose comprenant le stade de traitement du D-glucose dans un milieu aqueux avec une isomérase produite par un micro-organisme appartenant au genre *ampullariella.*

6. Procédé selon la revendication 5, dans lequel un agent de stabilisation thermique est présent pendant l'isomérisation.

7. Procédé selon la revendication 6, dans lequel l'agent de stabilisation thermique est un cation choisi parmi le groupe constitué par les ions cobalt, magnésium et manganèse.

8. Procédé selon la revendication 7, dans lequel la glucose-isomérase est immobilisée sur un support insoluble dans l'eau.

9. Procédé selon la revendication 8, dans lequel environ 1 kg (pound) de glucose-isomérase immobilisé est présent pour l'isomérisation d'environ 50 à 5000 kg (pounds) de mélange de glucose/fructose en tant que matières solides sèches.

10. Procédé selon la revendication 9, dans lequel le pH est d'environ 6 à environ 8,5, et la température est d'environ 45°C à environ 85°C.

11. Culture biologiquement pure d'un micro-organisme choisi parmi l'*ampullariella* espèces 3876, 3877, 3965, ou 3966 ayant les caractéristiques d'identification de ATCC 31351, 31352, 31353 ou 31354, respectivement, ladite culture étant capable de produire la glucose-isomérase en une quantité récupérable, par fermentation dans un milieu nutritif aqueux contenant des sources assimilables de carbone, d'azote et des produits minéraux essentiels.

12. Culture comprenant un micro-organisme choisi parmi l'*ampullariella* espèces 3876 ATCC 31351, 3877 ATCC 31352, 3965 ATCC 31353 ou 3966 ATCC 31354, ou un mutant de ces espèces et un milieu de culture nutritif, aqueux, contenant des sources assimilables de carbone, d'azote, et des produits minéraux essentiels, ladite culture étant capable de produire la glucose-isomérase en une quantité récupérable, par fermentation aérobie.

13. La culture selon la revendication 12, comprenant encore d'environ 0,1% à 10% en poids de xylose.

### Patentansprüche

1. Verfahren zur Herstellung von Glukose-Isomerase von einem Mikroorganismus aus der Gattung *Ampullariella,* welches das Züchten der *Ampullariella* auf einem Kulturmedium und die Gewinnung der Glukose-Isomerase daraus umfaßt.

2. Verfahren gemäß Anspruch 1, wobei das Kulturmedium Xylose oder eine Xylose-liefernde Quelle enthält.

3. Verfahren gemäß Anspruch 1 oder 2, wobei der Mikroorganismus aus *Ampullariella digitata, Ampullariella lobata, Ampullariella campanulata, Ampullariella regularis* oder eine Mutant derselben gewählt wird.

4. Verfahren gemäß Anspruch 1 oder 2, wobei der Mikroorganismus aus *Ampullariella*-Spezies 3876 (ATCC 31351), *Ampullariella*-Spezies 3877 (ATCC 31352), *Ampullariella*-Spezies 3965 (ATCC 31353), *Ampullariella*-Spezies 3966 (ATCC 31354) oder eine Mutant derselben gewählt wird.

5. Verfahren zur Isomerisierung der D-Glukose zu D-Fruktose einschließlich des Schritts zur Behandlung der D-Glukose in einem wäßrigen Medium mit einer isomerase, die von einem Mikroorganismus der Gattung *Ampullariella* erzeugt wird.

6. Verfahren gemäß Anspruch 5, wobei während der Isomerisierung ein wärmestabilisierendes Agens anwesend ist.

7. Verfahren gemäß Anspruch 6, wobei dieses wärmestabilisierende Agens ein Kation aus der Gruppe Kobalt-, Magnesium- und Manganionen ist.

8. Verfahren gemäß Anspruch 5, wobei die Glukose-Isomerase auf einem wasserunlöslichen Träger immobilisiert wird.

**0017656**

9. Verfahren gemäß Anspruch 8, wobei etwa ein Pfund (kg) der immobilisierten Glukose-Isomerase zur Isomerisation von etwa 50—5000 Pfund (kg) Glukose/Fruktosegemisch als trockene Feststoffe vorhanden sind.

10. Verfahren gemäß Anspruch 9, wobei der pH zwischen etwa 6 und etwa 8,5 und die Temperatur zwischen etwa 45°C und etwa 85°C liegt.

11. Eine biologisch reine Kultur eines Mikroorganismus, gewählt aus den *Ampullariella*-Spezies 3867, 3877, 3965 oder 3966, die die Identifizierungsmerkmale ATCC 31351, 31352, 31353 bzw. 31354 haben, wobei diese Kulturen in der Lage sind, nach Fermentation in einem wäßrigen Nährmedium mit assimilierbaren Quellen für Kohlenstoff, Stickstoff und wesentliche Mineralstoffe Glukose-Isomerase in gewinnbaren Mengen zu produzieren.

12. Eine Kultur, die einen Mikroorganismus, gewählt aus den *Ampullariella*-Spezies 3876 ATCC 31351, 3877 ATCC 31352, 3965 ATCC 31353 oder 3966 ATCC 31354 oder einer Mutation derselben und ein wäßriges Nährkulturmedium enthaltend assimilierbare Quellen für Kohlenstoff, Stickstoff und wesentliche Mineralstoffe umfaßt, wobei diese Kultur in der Lage ist, nach aerobischer Fermentierung Glukose-Isomerase in gewinnbaren Mengen zu produzieren.

13. Die Kultur gemäß Anspruch 12, die ferner etwa 0,1 bis etwa 10 Gewichtsprozent Xylose enthält.